# FASCICULE DE BREVET EUROPEEN

(11) **EP 0 513 139 B1**
(45) Date de publication et mention de la délivrance du brevet: **24.08.1994**
(21) Numéro de dépôt: 91903680.6
(22) Date de dépôt: 25.01.1991
(51) Int. Cl.: B01D 29/01, B01D 29/50, G01N 33/53, C12M 1/12

(54) **DISPOSITIF MODULAIRE POUR LE RECUEIL, L'INCUBATION, LA FILTRATION D'ECHANTILLONS MULTIPLES**
MODULARE VORRICHTUNG ZUM SAMMELN, INKUBIEREN UND FILTRIEREN VON MEHREREN PROBEN
MODULAR DEVICE FOR COLLECTING, INCUBATING AND FILTERING OF MULTIPLE SAMPLES

(30) Priorité: 26.01.1990 FR 9000959
(43) Date de publication de la demande: 19.11.1992
(73) Titulaire: BIOCOM, F-91942 Les Ulis Cédex (FR)
(72) Inventeur: BISCONTE, Jean-Claude, F-91640 Briis-sous-Forges (FR)
(74) Mandataire: Ores, Irène
(86) Numéro de dépôt international: FR9100044
(87) Numéro de publication internationale: WO9111245

(56) Documents cités:
- FR-A- 2 638 101
- GB-A-21 766 01
- US-A- 4 427 415
- US-A- 4 493 815
- US-A- 4 834 946
- US-A-37 303 52

## Description

La présente invention est relative à un dispositif modulaire pour le recueil, l'incubation, la filtration d'échantillons multiples.

L'utilisation des membranes filtrantes connaît une vogue croissante dans le cadre des applications d'analyse de particules, de cellules et de bactéries. Les membranes calibrées en polycarbonate produites par des Sociétés comme NUCLEOPORE et MILLIPORE ont des propriétés remarquables quant à leurs qualités de recueil de particules de tailles déterminées.

Très fines et résistances aux agents chimiques, elles se prêtent bien à l'observation sous microscope, soit en épi-éclairage, soit en éclairage transmis. Dans ces dernières conditions on est obligé de rendre transparente la membrane pour permettre l'observation. En éclairage épifluorescent, et à condition d'avoir coloré les cellules et les bactéries par un fluorochrome comme l'acridine orange, l'observation montre des particules claires sur un fond sombre.

En 1980 PETTIPHER a proposé cette méthode pour l'observation directe des bactéries du lait cru à des fins de contrôle de qualité. La composition complexe du lait et la présence de cellules en nombre élevé (plusieurs dizaines de milliers) rend impossible la filtration des échantillons sans un traitement préalable. Le prétraitement consiste à traiter le lait par un mélange de détergent et de trypsine. Dans des conditions précises de concentration et de temps, une désagrégation des micelles de caseine et des globules gras ainsi que la digestion des cellules sont obtenus sans modifier sensiblement le contenu en germes, qui peuvent alors étre colorés.

La filtration est pratiquée sur des filtres de taille importante et en dépression. Après différents rinçages on applique le colorant suivi d'autres rinçages. Cette méthode, dite méthode DEFT (Direct Epifluorescence Technique), présente de nombreux avantages. La filtration conduit à une importante concentration des événements à compter.

Par rapport aux méthodes indirectes, qui supposent une amplification de l'information bactérienne par prolifération (2 à 3 jours), les méthodes directes sont supposées donner un résultat rapide. La concentration par filtration peut aboutir à une excellente sensibilité si le milieu porteur est très filtrable.

Le handicap principal est la complexité de la méthode et de la sensibilité des résultats aux conditions de préparation et de mesure.

L'observation se fait sous microscope et, lorsque les densités d'événements sont faibles, les fluctuations de répartition sur le filtre obligent à des comptages de nombreux champs microscopiques. Par exemple, dans l'application de contrôle du lait cru pour des densités de l'ordre de 50 000 UFC/ml (UFC = Unités Formant Colonies), la méthode DEFT conduit à des répartitions de l'ordre de moins d'une bactérie par champ microscopique. Par ailleurs, les propriétés mécaniques très médiocres de la membrane de polycarbonate et sa finesse rendent très difficile l'observation sous microscope.

Placées entre lame et lamelle, les déformations conjuguées de la lame de verre, de la membrane et la platine du microscope expliquent l'échec des tentatives d'analyse d'image automatique. Malgré la présence de dispositifs de focalisation, les pertes de focus sont inévitables et une part prépondérante du temps d'analyse est consacrée à la recherche de la mise au point.

En 1988, BISCONTE proposait 2 solutions qui répondent en grande partie aux problèmes posés et qui font l'objet de deux Demandes de Brevets. Une première Demande de Brevet, FR-2 638 101, concerne un dispositif permettant de traiter automatiquement en parallèle plusieurs dizaines d'échantillons sur un même filtre. Une seconde Demande de Brevet, FR-2 638 240, est relative à la combinaison d'un moyen de contention des filtres ainsi qu'à leur placement sur une platine de microscope fonctionnant par aspiration

La première solution, qui intégre la présence des filtres ainsi montés dans des cadres support, apporte une solution à la question du passage en parallèle, simultané et indépendant, de plusieurs échantillons à filtrer, grâce à la mise en oeuvre simultanée de la pression et de la dépression. Dans cette solution, un récipient à puits multiples es mis en communication avec le filtre bar des tubes capillaires. Ces tubes permettent le passage successif des échantillons et des réactifs.

L'inconvénient principal de cette disposition est de rendre délicat le rinçage des canalisations (risque de contamination et résidus de colorants).

La seconde solution consiste principalement à monter les filtres dans un support semi-rigide indexé en position. Ce filtre se place d'abord dans le système de filtration selon une position rigoureuse grâce à des ergots de centrage. Des ergots présents sur le microscope garantissent un positionnement homologue pour l'observation. Ainsi les dépôts multiples présents sur le filtre, même de très petite taille, peuvent être retrouvés facilement par un programme informatique pilotant en X et Y la platine du microscope.

Les documents GB-A-2 176 601 et US-A-3 730 352 décrivent des dispositifs de filtration comprenant un ensemble de deux blocs supérieur et inférieur assemblés l'un à l'autre avec interposition d'un dispositif de filtrage équipé d'un filtre. Le bloc supérieur comporte des puits pour recevoir des échantillons à filtrer, et le bloc inférieur comporte des puits pour récupérer les filtrats. Enfin, ces dispositifs sont équipés de moyens de filtration forcée et de moyens d'évacuation des filtrats.

La présente invention vise à perfectionner les dispositifs de filtration précités pour permettre notamment une manipulation plus aisée des composants de ces dispositifs et augmenter les performances.

A cet effet, l'invention propose un dispositif modulaire pour le recueil, l'incubation et la filtration d'échantillons fluides, liquides ou gazeux, contenant des particules à filtrer, telles que bactéries, cellules ou autres éléments, notamment contenues dans le lait ou le sang, du type comprenant :
- un bâti ;
- au moins un filtre monté entre deux blocs de serrage respectivement amont et aval, en considérant le sens de filtration, destinés à être fixés l'un à l'autre avec interposition d'un joint d'étanchéité perforé, le bloc de serrage amont comprenant un ensemble de conduits parallèles formant des puits de stockage des échantillons à traiter, alors que le bloc de serrage aval est réalisé sous la forme d'un plateau perforé, les puits, les perforations du joint et les perforations du plateau étant mutuellement en regard pour définir autant de zones de filtration indépendantes au niveau du filtre ;
- des moyens de filtration forcée, et
- des moyens d'évacuation des filtrats ; caractérisé en ce que :
- le bloc de serrage amont est un bloc-réservoir indépendant permettant de procéder à un traitement préalable, tel qu'une incubation des échantillons contenus dans les puits, ce traitement étant effectué à l'extérieur du bâti ;
- le bloc de serrage amont est fixé, en dehors du bâti, au bloc de serrage aval par des moyens de fixation complémentaires détachables l'un de l'autre ; et
- l'ensemble des deux blocs de serrage est logé de façon amovible à l'intérieur du bâti en étant introduit par une ouverture latérale de celui-ci, pour procéder à la filtration des échantillons prétraités, ledit bâti comportant une ouverture supérieure pour y monter les moyens de filtration forcée.

Selon une autre caractéristique de l'invention, pour procéder au traitement préalable des échantillons dans le bloc-réservoir et en dehors du bâti, il est prévu au moins un moyen d'obturation amovible des puits à la base inférieure du bloc-réservoir, le bloc de serrage aval est ensuite rapporté et fixé sur la base supérieure du bloc-réservoir et l'ensemble des deux blocs est retourné avant d'être logé dans le bâti.

D'une manière générale et selon une autre caractéristique de l'invention, l'ensemble des deux blocs est logé à l'intérieur du bâti à la façon d'un tiroir au moyen de glissières ménagées dans l'encadrement de l'ouverture latérale du bâti, et les moyens de fixation des deux blocs de serrage sont constitués par des languettes d'encliquetage destinées à coopérer avec des ergots.

Selon une autre caractéristique de l'invention, la base aval du bloc-réservoir, la base amont du bloc de serrage aval et le filtre sont pourvus de moyens d'indexation complémentaires assurant l'alignement entre leurs perforations respectives.

Selon encore une autre caractéristique de l'invention, les moyens de filtration forcée sont constitués par des moyens de mise sous pression des échantillons contenus dans les puits du bloc de serrage amont, lesdits moyens étant supportés par une tête constituée d'une partie fixe et d'une partie mobile comprenant une chambre de mise sous pression dont le plancher est traversé par une pluralité de tubes qui sont calibrés et destinés à être introduits partiellement dans les puits du bloc de serrage amont en étant en communication avec une arrivée d'air sous pression ménagée dans le plafond de la chambre, le diamètre externe des tubes calibrés étant sensiblement égal au diamètre interne des puits, ladite partie mobile étant déplaçable par l'intermédiaire d'un moyen d'actionnement, tel qu'un vérin.

Selon un exemple de réalisation, le plafond de la chambre de mise sous pression est traversé par une pluralité de tubes qui passent à l'intérieur des tubes calibrés du plancher de ladite chambre, lesdits tubes étant en communication avec un dispositif d'alimentation pour introduire des agents de traitement, et le moyen d'obturation des puits du bloc de serrage amont est constitué par une membrane adhésive jetable, et les tubes calibrés des moyens de filtration forcée sont biseautés pour percer ladite membrane.

D'une manière générale et selon une autre caractéristique de l'invention, les moyens d'évacuation sont constitués par un réservoir monté dans la partie inférieure du bâti, le bloc de serrage aval s'appliquant de manière étanche sur ledit réservoir lorsqu'il est introduit dans le bâti, et ledit réservoir d'évacuation communique avec une ouverture prévue à la face inférieure du bâti.

Dans le cas où une double filtration s'avère nécessaire pour les échantillons, le dispositif selon l'invention comporte deux filtres de porosités différentes placés de part et d'autre du joint perforé, le filtre présentant des pores de moindre diamètre étant disposé en aval.

En variante, dans le cas où une double filtration s'avère nécessaire, les moyens d'évacuation sont amovibles et constitués par un bloc qui est identique au bloc-réservoir et qui comporte un moyen d'obturation amovible de sa base aval, ces moyens d'évacuation formant réservoir étant destinés a' remplacer le bloc-réservoir amont et à être remplacés par des moyens d'évacuation normaux après la première filtration de manière à effectuer la deuxième filtration sur un deuxième filtre remplaçant le premier filtre porté par le tiroir de filtration.

Selon une variante préférée de l'invention, dans le cas où une double filtration s'avère nécessaire pour les échantillons, ce dispositif comporte un deuxième bloc-réservoir et un deuxième tiroir de filtration, entre lesquels sont interposés un deuxième joint perforé et un deuxième filtre et qui sont disposés en cascade par rapport au premier bloc-réservoir et au premier tiroir de filtration, le bâti comportant deux autres glissières horizontales destinées à y permettre l'introduction du deuxième tiroir de filtration avec le deuxième bloc-réservoir qu'il supporte.

Avantageusement, le tiroir de filtration est destiné à coopérer avec une pluralité de blocs-réservoirs identiques entre eux dont le nombre est fonction du nombre d'échantillons.

Selon une autre caractéristique de l'invention, les moyens de filtration forcée comprennent aussi des moyens d'aspiration des filtrats communiquant avec les moyens d'évacuation et coopérant avec les moyens de filtration forcée par mise sous pression.

Enfin, selon encore une autre caractéristique de l'invention, le dispositif est à fonctionnement automatique et programmable, et en ce que les moyens de filtration forcée, à savoir de mise sous pression et/ou d'aspiration sont eux aussi programmables, l'ensemble étant sous le contrôle d'un ordinateur de pilotage.

Outre les dispositions qui précédent, l'invention comprend encore d'autres dispositions, qui ressortiront de la description qui va suivre.

L'invention sera mieux comprise à l'aide du complément de description qui va suivre, qui se réfère aux dessins annexés, dans lesquels :
- la figure 1 est une vue schématique en perspective du dispositif de filtration selon l'invention ;
- la figure 2 est une vue en coupe, également schématique et suivant II, du dispositif illustré à la figure 1.

Il doit être bien entendu, toutefois, que ces dessins et les parties descriptives correspondantes, sont donnés uniquement à titre d'illustration de l'objet de l'invention, dont ils ne constituent en aucune manière une limitation.

L'originalité de la solution proposée dans la présente invention consiste essentiellement en ce qu'elle permet de réunir dans un élément unique la fonction de traitement des échantillons, préalable à la filtration (tel que recueil, incubation, digestion, etc...) et en dehors du dispositif de filtration, et la fonction de traitement des particules de chaque échantillon retenues par le filtre après la filtration (tel que rinçage, coloration, séchage, etc...) et à l'intérieur même du dispositif de filtration : il s'agit du bloc-réservoir.

Le concept du bloc-réservoir amovible a les avantages suivants :
- on peut utiliser une pluralité de blocs de ce type présentant des puits (ou conduits) ouverts aux deux bouts. Ils sont donc simples, faciles à nettoyer et peuvent être très nombreux pour alimenter le dispositif de filtration et de coloration en fonction du nombre d'échantillons et d'un protocole d'analyse prédéterminé ;
- après la filtration les puits servent à canaliser le passage de réactifs vers le filtre. Il y a donc dissociation complète entre les circuits contenant les échantillons et les circuits de réactifs, ce qui élimine les risques de contamination.

De la même façon, le joint d'étanchéité est amovible et peut être remplacé aisément.

Le principe est le suivant : des blocs parallélipédiques (ou ayant une autre forme) sont percés de 24 trous cylindriques parallèles (ou convergents) ouverts aux deux bouts. On place sur l'une des faces une plaque d'obturation porteuse de 24 bouchons souples (type caoutchouc). Des membranes adhésives jetables peuvent également être utilisées. On peut ainsi garnir les 24 puits d'échantillons par un distributeur automatique (non représenté), par exemple du type commercialisé sous les noms de marques de fabrique TECAN^{(R)} ou GILSON^{(R)}, ou encore par un système manuel type multipipettes standard (dans le cas présenté l'espacement de 9 mm entre les puits est une disposition classique).

La distribution peut concerner des dizaines de blocs-réservoirs qui peuvent ensuite être traités par des machines automatiques. Selon le temps de latence entre le remplissage et la phase de filtration, on peut placer une plaque d'obturation non seulement au niveau de la base inférieure, mais aussi au niveau de la base supérieure pour éviter les évaporations et les contaminations.

L'autre point important est que le filtre est placé dans un deuxième bloc perforé faisant tiroir de filtration, lui-même amovible. Ce tiroir, préalablement garni du filtre et d'un joint d'étanchéité, est placé de façon étanche, en dehors du dispositif de filtration, contre le bloc-réservoir, sur sa face supérieure, après avoir enlevé l'éventuelle plaque d'obturation correspondante. A cette phase ce bloc est ainsi fermé sur ses deux faces, d'une part, par la plaque d'obturation inférieure et, d'autre part, par le tiroir porteur du filtre sur la face supérieure. On peut alors retourner le bloc-réservoir et l'introduire dans le dispositif de filtration.

Dans une configuration très simple il suffit d'appliquer une aspiration sous le tiroir de filtration et d'enlever la plaque d'obturation inférieure qui se retrouve à la partie supérieure après avoir retourné le bloc-réservoir. Ci-après, on se réfère plus particulièrement à un dispositif de filtration plus complexe permettant d'appliquer automatiquement une pression en amont du filtre et, le cas échéant, une dépression en aval et qui comprend un distributeur, de préférence automatique (non représenté), de type connu des techniciens en la matière, chargé d'alimenter en réactifs le bloc-réservoir après la filtration des échantillons.

Ci-après on donne une description générale succinte de ce dispositif de filtration, qui comprend les éléments principaux suivants :
. un bâti (ou une enceinte) 1 : il est destiné a supporter de façon rigide les différents composants ;
. un tiroir de filtration 2 : il est destiné à supporter le(s) filtre(s) et les blocs-réservoirs l'un après l'autre ;
. une tête mobile 3 : actionnée par un vérin 4, elle permet d'injecter les réactifs et les fluides gazeux, tout en permettant d'effectuer la filtration forcée par mise sous pression des échantillons ;
. un bloc-réservoir à fonctions multiples 5 : ce bloc contient d'abord les échantillons à filtrer et ensuite les réactifs ;
. un dispositif de vidange 6 : il recueille les filtrats et permet leur évacuation ;
. un joint perforé 7 extractible : il est placé entre le bloc-réservoir et le tiroir de filtration, lui-même porteur du filtre 13 ;
. un filtre 13 : le filtre est serré entre le tiroir 2 et le joint perforé 7.

Cette description générale est suivie d'une description détaillée des différents composants.

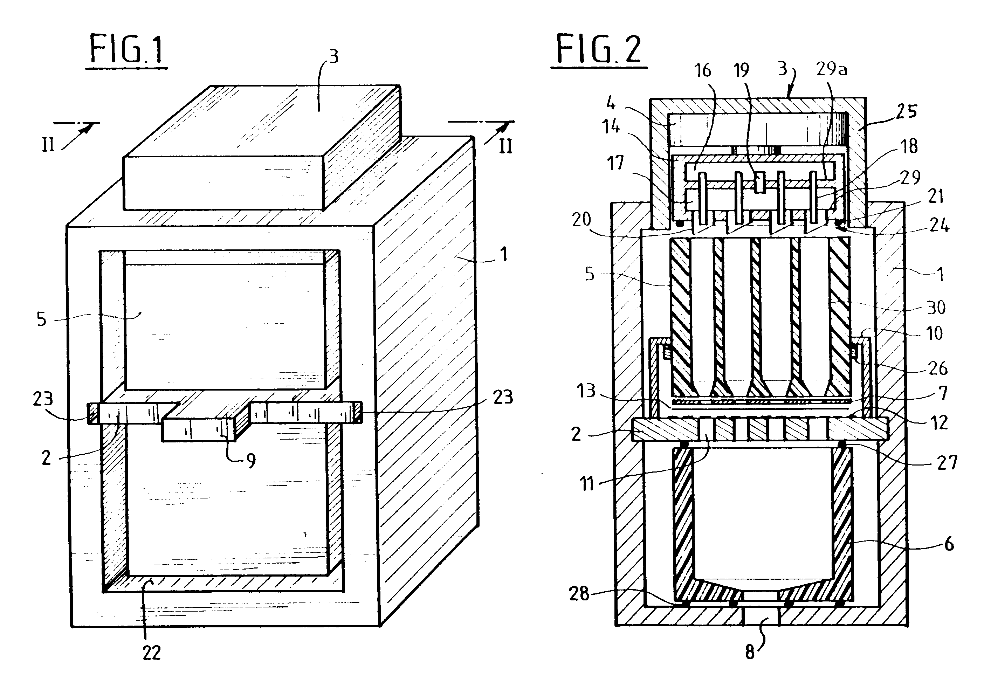

Le bâti 1 : usiné en alliage léger, le bâti se présente comme un volume parallépipédique ouvert sur l'avant (cf l'ouverture 22 à la figure 1). Il porte en partie haute la tête mobile 3, qui accède à l'intérieur du dispositif par l'ouverture 24, et deux rainures ou glissières horizontales 23 qui sont ménagées dans les parois latérales verticales du bâti 1 et dans lesquelles glisse le tiroir de filtration 2. En partie basse un crifice 8 permet l'aspiration des filtrats à l'aide d'un dispositif d'aspiration (non représenté) communiquant avec le dispositif de vidange 6.

Le tiroir de filtration 2 : est usiné en acier inoxydable ou tout autre matériau rigide résistant à la corrosion chimique. Ce tiroir comprend une tirette 9 placée en face avant destinée à faciliter l'introduction et l'extraction du tiroir.

Le tiroir porté deux languettes inoxydables 10 qui permettent de fixer fermement le bloc-réservoir en position. Le tiroir est percé de 24 trous calibrés 11. Les perforations de ce tiroir sont fermées à la partie supérieure par un grillage 12 sur lequel vient en appui le filtre 13. Il porte des ergots (non représentés) qui traversent le filtre et le joint et viennent se loger dans le bloc-réservoir 5 assurant l'alignement de ces composants.

La tête mobile 3 comprend elle-même une partie fixe 25 fixée au bâti 1 et une partie mobile proprement dite 14 capable de se mouvoir de bas en haut sous l'action d'un vérin 4. La tête mobile comprend un espace 16 dans lequel émergent 24 tubes d'alimentation 18 destinés à injecter les réactifs dans les 24 puits du bloc 5, après avoir été reliés à un dispositif de distribution automatique (non représenté). Une arrivée d'air comprimé 19 permet de mettre en pression une chambre 17. Cette chambre comprend 24 tubes bisautés 20 fixés à son plancher 29 et contenant en leur centre les tubes d'alimentation 18 fixés au plafond 29a de cette chambre. La tête mobile comprend un joint 21 destiné à créer l'étanchéité avec le bloc 5.

Le bloc-réservoir 5 se présente comme un bloc parallépipédique métallique ou en matériau synthétique (suivant les usages). Il est percé de 24 trous ou conduits 30 débouchant aux deux extrémités ; la partie de ces trous placée vers le filtre est calibrée et conique. Des ergots 26 placés latéralement sont agrippés par les languettes 10 du tiroir de filtration 2.

Le bloc-réservoir comporte des évidements (non représentés) destinés à aligner et détromper l'emplacement de tous les éléments, comme le filtre et le joint, conjointement avec le tiroir de filtration, ainsi que déjà évoqué plus haut.

Le dispositif de vidange 6 est un réservoir réalisé en matériau léger. Il a de préférence les mêmes dimensions extérieurement qu'un bloc-réservoir. Il est muni de joints d'étanchéité aussi bien en partie haute qu'en partie basse, 27 et 28.

Le joint perforé et extractible 7 est une plaque en matériau souple, écrasable, percée de 24 trous. Deux trous (non représentés) sont traversés par les ergots du tiroir de filtration et assurent ainsi l'alignement entre les 24 orifices du bloc 5 et les 24 orifices du tiroir de filtration 2, conformément à la disposition précitée.

Le filtre 13 est du type utilisé dans le cadre de la Demande de Brevet FR-2 638 101. Il comprend une membrane filtrante tendue dans un cadre plastique semi-rigide. Des évidements (non représentés) permettent le passage des ergots d'alignement précités du tiroir de filtration.

En ce qui concerne la représentation schématique du dispositif de filtration à la figure 2, il y a lieu de remarquer que le bloc-réservoir 5 ainsi que le joint perforé 7 et le filtre 13 sont représentés séparés du tiroir de filtration 2 pour des raisons d'intelligibilité du dessin, mais il est clair que tous ces éléments sont en contact serré entre eux.

Ci-après, aux points 1 à 4, est décrit le fonctionnement du dispositif de filtration.
**1.** Utilisation du bloc-réservoir : le bloc-réservoir 5 est destiné à recevoir successivement les échantillons à filtrer, les réactifs de rinçage, les colorants et les gaz comme l'air destinés à chasser les liquides ou encore à sécher le(s) filtre(s). On peut l'utiliser avant ou après la fixation sur le tiroir. Il peut également être utilisé comme un récipient d'incubation.
   * Utilisation préalable à la fixation sur le tiroir : le bloc-réservoir 5 peut être muni de plaques d'oburation (non représentées) en face supérieure et inférieure. On peut ainsi fermer une extrémité, placer les échantillons dans les puits 30 pour les stocker ou incuber ceux-ci. Une plaque d'obturation supérieure empêche l'évaporation ou la contamination. Pour passer aux étapes de filtration et de coloration, ce bloc-réservoir est placé à l'envers, (face supérieure en bas). La plaque d'obturation supérieure est enlevée tandis que la plaque inférieure est maintenue sur l'autre face.

   Le tiroir de filtration 2 ayant été garni préalablement du filtre 13, puis du joint perforé 7, est retourné et plaqué contre le bloc 5. Une pression ferme écrase le joint perforé 7 et les languettes 10 viennent s'encliqueter sur les ergots 26 du bloc 5. Le tiroir portant l'ensemble ainsi fixé rigidement est retourné, la plaque d'obturation est enlevée et le tiroir peut être introduit dans les glissières 23 du bâti 1.
   * Utilisation directe : le tiroir de filtration est dégagé aux 3/4 du bâti, on place le filtre et le joint comme précédemment, puis le bloc-réservoir est encliqueté en position normale, les plaques d'obturation étant toutes enlevées.

   On peut alors introduire les échantillons, le filtre s'opposant à un passage prématuré en dehors de toute application de pression et/ou de dépression (ceci ne vaut que pour des filtres à pores de petite dimension). On repousse ensuite le tiroir et le processus peut commencer. On peut également enfoncer le tiroir avant d'avoir rempli les puits. Le processus de remplissage s'effectue alors par les tubes de remplissage 18, de façon automatique ou non, lorsqu'il n'y a pas lieu de craindre des risques de contamination.
   Le bloc-réservoir à fonctions multiples peut recevoir des milieux variés liquides ou gazeux. Une utilisation particulière peut être faite dans une application biotechnologique.
   * Utilisation en biotechnologie : le bloc-réservoir stérilisé reçoit comme précédemment un filtre et se trouve placé sur le tiroir. Une suspension cellulaire est introduite dans les puits. Placé dans un incubateur, les cellules peuvent proliférer grâce au milieu nutritif de la suspension. Le filtre tend alors a être recouvert d'un tapis cellulaire. Les arrivés différentes pour chaque puits permettent d'introduire, de façon automatique ou non, des substances à tester. Inversement, au-dessous du filtre, des prélèvements réguliers peuvent être effectués afin d'identifier des substances produites par les cellules. Au bout d'un temps de culture variable on peut retirer le tiroir, retourner le bloc-réservoir pour évacuer les liquides et retirer le filtre. On peut ainsi prélever des cellules vivantes ou conduire un processus complet de culture avec à terme une fixation, une coloration et une observation automatique des résultats. Ceci constitue un moyen très rapide et simple de tester des molécules quant à leur effet sur la prolifération et la différenciation cellulaire.
**2.** Utilisation du tiroir de filtration : Ce tiroir, qui est indépendant du bâti, à savoir amovible, permet la fixation du filtre, du joint et du bloc-réservoir. Introduit dans les glissières du bâti, le tiroir engagé à fond positionne automatiquement le bloc-réservoir face aux orifices d'arrivée des réactifs et de la pression. Dans une variante de configuration le tiroir peut être muni de raccords à chacune des ouvertures : on peut ainsi recueillir individuellement les filtrats ou encore suivre chacune des filtrations. Il suffit pour cela de faire aboutir chacun des tuyaux souples dans un tube de recueil à essai. Dans une autre variante préférée, tous les tubes de recueil sont placés dans une boîte étanche dans laquelle on peut faire le vide.
**3.** Description d'une séquence complète. Application à l'analyse cellulaire et bactérienne du lait cru (méthode DEFT) : Un distributeur automatique injecte dans les puits du bloc-réservoir une quantité déterminée d'un échantillon de lait cru additionné d'un mélange de Triton^{(R)} et du trypsine. Dans cette phase chaque bloc est fermé à sa partie inférieure par une plaque d'obturation (couvercle à bouchons multiples). Les puits sont fermés aussi à la partie supérieure et placés en incubation. Ce traitement a pour effet de rendre le lait plus filtrable. Les volumes traités sont de l'ordre de 200 »l de lait et autant de réactifs. Le volume de chaque puits est de 3 ml. Chacun des blocs après incubation est renversé et ouvert au niveau de la face de filtration. Le tiroir, garni du filtre et du joint, est encliqueté avec un bloc-réservoir. Dans ce cas il y a lieu de noter que le filtre est une membrane de polycarbonate de 0,6 »m destinée à retenir les germes bactériens. Après introduction du tiroir et enlèvement de la plaque d'obturation supérieure du bloc-réservoir, la séquence commence. Il faut noter que l'appareil réalisé comprend un environnement automatisé capable de pomper automatiquement des liquides et d'alterner des mises en pression et/ou en dépression avec des ajouts de liquides (cette façon particulière de procéder fait l'objet d'une description notamment dans la Demande de Brevet FR-2 638 101).
   Mise en étanchéité : le vérin 4 (notamment pneumatique) est actionné ; il abaisse la partie mobile 14 de la tête 3 qui vient s'appliquer très fermement contre le bloc-réservoir 5 par l'intermédiaire d'un joint 21. Il faut noter que les jeux permettent de transmettre ces efforts au bloc 5 qui vient ainsi s'appliquer en écrasant le joint perforé 7. Ceci garantit que les voies resteront indépendantes et qu'il n'y aura pas de mélange intempestif d'échantillons au cours de la filtration.
   Filtration du lait : un ordinateur programmé de pilotage du dispositif de filtration met en pression les puits par l'intermédiaire de l'arrivée de pression 19, cette pression s'appliquant à la chambre 17 qui la transmet par les tubes bisautés 20. Tous les puits se vident ensemble avec des différences peu significatives. Il y a lieu de noter qu'une dépression peut être appliquée dans le récipient de vidange 6 afin d'augmenter le gradient. La phase de vidange prend de 5 à 10 secondes.
   Rinçage et coloration : plusieurs réactifs se succèdent alors. Ils sont apportés en parallèle dans les 24 tuyaux 18. Ceux-ci sont placés en ligne directe avec les puits et pénètrent d'environ 1 cm dans ceux-ci.
   Se succèdent ainsi des milieux de rinçage, des tampons pH et enfin un colorant de fluorescence, l'acridine orange.
   Les volumes sont de l'ordre de 100 »l par réactif et la somme des réactifs et de l'échantillon est calculée de telle sorte que, même en cas de colmatage accidentel du filtre, le volume total reste inférieur à la capacité d'un puits, soit 3 ml.
   La programmation du dispositif est faite de telle sorte que le colorant reste au contact durant 2 minutes avec le filtre et les bactéries déposées. Après un ultime rinçage et la vidange, on fait passer de l'air pendant 2 minutes pour sécher totalement le filtre. Le tiroir 2 peut alors être retiré avec le bloc-réservoir 5, les languettes 10 dégagées et le bloc 5 enlevé, puis le filtre 13.
   On place alors le filtre sur une platine spéciale d'observation, telle que décrite dans la Demande de Brevet FR-2 638 240.
**4.** Cas de la double filtration : dans certains cas on peut avoir avantage à recueillir 2 types différents de particules ou de cellules. On peut ainsi placer deux filtres de porosités très différentes de part et d'autre du joint perforé 7. Ces filtres sont ainsi séparés de 2 mm et ne sont pas en contact.
   On peut également pratiquer la double filtration en séquence. Dans ce cas le récipient (6) est remplacé par un bloc identique au bloc 5 qui est simplement obturé sur sa face de filtration. Les filtrats se répartissent alors de façon homogène dans les puits. Dans une autre variante avantageuse on peut imaginer que le deuxième bloc 5 soit également muni d'un filtre et d'un tiroir de filtration. Dans ces conditions la pression est appliquée au bloc supérieur et une dépression au bloc inférieur (le dispositif de mise en dépression n'a pas été représenté). Bien entendu, une modification du bâti serait nécessaire pour créer deux autres glissières pour le deuxième tiroir de filtration.
   En particulier, il doit être bien entendu que, bien que cela ne soit pas indispensable, il est avantageux que le dispositif d'alimentation des produits de traitement des particules retenues par les zones de filtration indépendantes soit automatique et programmable ; de même les moyens de filtration forcée, à savoir de mise sous pression et/ou d'aspiration peuvent être eux aussi programmables, l'ensemble étant sous le contrôle d'un ordinateur de pilotage.

## Revendications

1. Dispositif modulaire pour le recueil, l'incubation et la filtration d'échantillons fluides, liquides ou gazeux, contenant des particules à filtrer, telles que bactéries, cellules ou autre éléments, notamment contenues dans le lait ou le sang, du type comprenant :
- un bâti (1);
- au moins un filtre (13) monté entre deux blocs de serrage respectivement amont (5) et aval (2), en considérant le sens de filtration, destinés à être fixés l'un à l'autre avec interposition d'un joint d'étanchéité perforé (7), le bloc de serrage amont (5) comprenant un ensemble de conduits parallèles formant des puits (30) de stockage des échantillons à traiter, alors que le bloc de serrage aval (2) est réalisé sous la forme d'un plateau perforé, les puits (30), les perforations du joint (7) et les perforations du plateau étant mutuellement en regard pour définir autant de zones de filtration indépendantes et étanches au niveau du filtre (13) ;
- des moyens de filtration forcée (17,19), et
- des moyens d'évacuation (6) des filtrats ;
caractérisé en ce que :
- le bloc de serrage amont (5) est un bloc-réservoir indépendant permettant de procéder à un traitement préalable, tel qu'une incubation des échantillons contenus dans les puits (30), ce traitement étant effectué à l'extérieur du bâti ;
- le bloc de serrage amont (5) est fixé, en dehors du bâti (1), au bloc de serrage aval (2) par des moyens de fixation complémentaires (10,26) détachables l'un de l'autre ; et
- l'ensemble des deux blocs de serrage (2,5) est logé de façon amovible à l'intérieur du bâti (1) en étant introduit par une ouverture latérale (22) de celui-ci, pour procéder à la filtration des échantillons prétraités, ledit bâti (1) comportant une ouverture supérieure (24) pour y monter les moyens de filtration forcée (17,18,19).

2. Dispositif selon la revendication 1, caractérisé en ce que, pour procéder au traitement préalable des échantillons dans le bloc-réservoir (5) et en dehors du bâti (1), il est prévu au moins un moyen d'obturation amovible des puits (30) à la base inférieure du bloc-réservoir (5), le bloc de serrage aval (2) est ensuite rapporté et fixé sur la base supérieure du bloc-réservoir (5), et l'ensemble des deux blocs (2,5) est retourné avant d'être logé dans le bâti (1).

3. Dispositif selon la revendication 1 ou 2, caractérisé en ce que l'ensemble des deux blocs (2,5) est logé à l'intérieur du bâti (1) à la façon d'un tiroir au moyen de glissières (23) ménagées dans l'encadrement de l'ouverture latérale (22) du bâti (1).

4. Dispositif selon l'une quelconque des revendications 1 à 3, caractérisé en ce que les moyens de fixation des deux blocs de serrage (2,5) sont constitués par des languettes d'encliquetage (10) destinées à coopérer avec des ergots (26).

5. Dispositif selon l'une quelconque des revendications 1 à 4, caractérisé en ce que la base aval du bloc-réservoir (5), la base amont du bloc de serrage aval (2) et le filtre (13) sont pourvus de moyens d'indexation complémentaires assurant l'alignement entre leurs perforations respectives.

6. Dispositif selon l'une quelconque des revendications 1 à 5 caractérisé en ce que les moyens de filtration forcée (17,18,19) sont constitués par des moyens de mise sous pression des échantillons contenus dans les puits (30) du bloc de serrage amont (5), lesdits moyens étant supportés par une tête (3) constituée d'une partie fixe (25) et d'une partie mobile (14) comprenant une chambre de mise sous pression (17) dont le plancher (29) est traversé par une pluralité de tubes (20) qui sont calibrés et destinés à être introduits partiellement dans les puits (30) du bloc de serrage amont (5) en étant en communication avec une arrivée d'air sous pression (19) ménagée dans le plafond (29a) de la chambre (17), le diamètre externe des tubes calibrés (20) étant sensiblement égal au diamètre interne des puits (30), ladite partie mobile (14) étant déplaçable par l'intermédiaire d'un moyen d'actionnement, tel qu'un vérin (4).

7. Dispositif selon la revendication 6, caractérisé en ce que le plafond (29a) de la chambre (17) de mise sous pression est traversé par une pluralité de tubes (18) qui passent à l'intérieur des tubes calibrés (20) du plancher (29) de ladite chambre (17), lesdits tubes (18) étant en communication avec un dispositif d'alimentation pour introduire des agents de traitement.

8. Dispositif selon la revendication 7, caractérisé en ce que le moyen d'obturation des puits (30) du bloc de serrage amont (5) est constitué par une membrane adhésive jetable, et en ce que les tubes calibrés (20) des moyens de filtration forcée (17,18,19) sont biseautés pour percer ladite membrane.

9. Dispositif selon l'une quelconque des revendications 1 à 8, caractérisé en ce que les moyens d'évacuation (6) sont constitués par un réservoir monté dans la partie inférieure du bâti (1), le bloc de serrage aval (2) s'appliquant de manière étanche sur ledit réservoir lorsqu'il est introduit dans le bâti (1).

10. Dispositif selon la revendication 9, caractérisé en ce que ledit réservoir d'évacuation communique avec une ouverture (8) prévue à la face inférieure du bâti (1).

11. Dispositif selon l'une quelconque des revendications précédentes, caractérisé en ce que, dans le cas où une double filtration s'avère nécessaire pour les échantillons, ce dispositif comporte deux filtres (13) de porosités différentes placés de part et d'autre du joint perforé (7), le filtre présentant des pores de moindre diamètre étant disposé en aval.

12. Dispositif selon l'une quelconque des revendications 1 à 9, caractérisé en ce que, dans le cas où une double filtration s'avère nécessaire, les moyens d'évacuation (6) sont amovibles et constitués par un bloc qui est identique au bloc-réservoir (5) et qui comporte un moyen d'obturation amovible de sa base aval, ces moyens d'évacuation faisant réservoir étant destinés à remplacer le bloc-réservoir amont (5) et à être remplacés par des moyens d'évacuation normaux (6) après la première filtration de manière à effectuer la deuxième filtration sur un deuxième filtre remplaçant le premier filtre porté par le tiroir de filtration (2).

13. Dispositif selon l'une quelconque des revendications 1 à 9, caractérisé en ce que, dans le cas où une double filtration s'avère nécessaire pour les échantillons, ce dispositif comporte un deuxième bloc-réservoir (5) et un deuxième tiroir de filtration (2), entre lesquels sont interposés un deuxième joint perforé (7) et un deuxième filtre (13) et qui sont disposés en cascade par rapport au premier bloc-réservoir (5) et au premier tiroir de filtration (2), le bâti (1) comportant deux autres glissières horizontales (23) destinées à y permettre l'introduction du deuxième tiroir de filtration (2) avec le deuxième bloc-réservoir qu'il supporte.

14. Dispositif selon l'une quelconque des revendications précédentes, caractérisé en ce que le tiroir de filtration (2) est destiné à coopérer avec une pluralité de blocs-réservoirs (5) identiques entre eux dont le nombre est fonction du nombre d'échantillons.

15. Dispositif selon l'une quelconque des revendications précédentes, caractérisé en ce que les moyens de filtration forcée comprennent aussi des moyens d'aspiration des filtrats communiquant avec les moyens d'évacuation (6) et coopérant avec les moyens de filtration forcée par mise sous pression (17,18,19).

16. Dispositif selon l'une quelconque des revendications précédentes, caractérisé en ce que ledit dispositif est à fonctionnement automatique et programmable, et en ce que les moyens de filtration forcée, à savoir de mise sous pression et/ou d'aspiration sont eux aussi programmables, l'ensemble étant sous le contrôle d'un ordinateur de pilotage.

## Claims

1. A modular device for collecting, incubating, and filtering liquid or gaseous fluid samples containing particles to be filtered such as bacteria, cells, or other elements, in particular those contained in milk or in blood, the device being of the type comprising:
- a stand (1);
- at least one filter (13) mounted between two clamping blocks that are respectively upstream (5) and downstream (2) in the filtering direction, the blocks being designed to be fixed one against the other with an interposed perforated gasket (7), the upstream clamping block (5) including a plurality of well-forming parallel ducts for storing samples to be treated, whereas the downsteam clamping block (2) is implemented in the form of a perforated tray, the wells (30), the perforations through the gasket (7), and the perforations through the tray being mutually in register to define as many mutually sealed independent filter zones on the filter (13);
- forced filtering means (17, 19); and
- filtrate evacuation means (6); the device being characterized in that:
- the upstream clamping block (5) is an independent tank-block enabling pretreatment to be performed, such as incubation of the samples contained in the wells (20), which treatment is performed outside the stand;
- outside the stand (1), the upstream clamping block (5) is fixed to the downstream clamping block (2) by additional fixing means (10, 26) that are removable from one another; and
- the set of two clamping blocks (2, 5) is removably received inside the stand (1) by being inserted through a lateral opening (22) thereof, to proceed with filtering of the pretreated samples, said stand (1) including a top opening (24) for receiving the forced filtering means (17, 18, 19).

2. A device according to claim 1, characterized in that in order to perform pretreatment of the samples in the tank-block (5), outside the frame (1), at least one removable well-closure means is provided at the bottom base of the tank-block (5), the downstream clamping block (2) is then applied and fixed to the top base of the tank block (5), and the two-block assembly (2, 5) is turned over prior to being housed in the stand (1).

3. A device according to claim 1 or 2, characterized in that the two-block set (2, 5) is housed inside the stand (1) like a drawer by means of slideways (23) formed in the framing of the lateral opening (22) of the stand (1).

4. A device according to any one of claims 1 to 3, characterized in that the means for fixing the two clamping blocks (2, 5) are constituted by snap-fastening tongues (10) designed to cooperate with studs (26).

5. A device according to any one of claims 1 to 4, characterized in that the downstream base of the tank-block (5), the upstream base of the downstream clamping block (2), and the filter (13) are all provided with complementary indexing means ensuring that their respective perforations are in alignment.

6. A device according to any one of claims 1 to 5, characterized in that the forced filtering means (17, 18, 19) are constituted by means for putting the samples contained in the wells (30) of the upstream clamping block (5) under pressure, said means being supported by a head (3) constituted by a stationary portion (25) and by a moving portion (14) comprising a pressurizing chamber (17) whose floor (29) has a plurality of tubes (20) passing therethrough, which tubes are calibrated and designed to be inserted partially into the wells (30) of the upstream clamping block (5) while being in communication with an inlet (19) for air under pressure provided in the ceiling (29a) of the chamber (17), the outside diameter of the calibrated tubes (20) being substantially equal to the inside diameter of the wells (30), said moving portion (14) being displaceable under drive from actuator means such as an actuator (4).

7. A device according to claim 6, characterized in that the ceiling (29a) of the pressurizing chamber (17) has a plurality of tubes (18) passing therethrough which pass inside the calibrated tubes (20) of the floor (29) of said chamber (17), said tubes (20) being in communication with a feed device for feeding treatment agents.

8. A device according to claim 7, characterized in that the means for closing the wells (30) of the upstream clamping block (5) is constituted by a discardable adhesive membrane, and in that the calibrated tubes (20) of the forced filtering means (17, 18, 19) are chamfered to pierce said membrane.

9. A device according to any one of claims 1 to 8, characterized in that the emptying means (6) are constituted by a tank mounted in the bottom portion of the stand (1), the downsteam clamping block (2) bearing in field manner against said tank when it is inserted in the stand (1).

10. A device according to claim 9, characterized in that said emptying tank communicates with an opening (8) provided in the bottom face of the stand (1).

11. A device according to any preceding claim, characterized in that, in the event that double filtering is necessary for the samples, the device includes two filters (13) having different pore sizes placed on opposite sides of the perforated gasket (7), the filter having the smaller-diameter pores being placed downstream.

12. A device according to any one of claims 1 to 9, characterized in that, in the event that double filtering is necessary, the evacuation means are removable and are constituted by a block which is identical to the tank-block (5) and which includes removable closure means for its downstream face, said evacuation means constituting a tank being intended, after the first filtering operation, to replace the upstream tank-block (5) and to be replaced by normal evacuation means (6) so as to enable the second filtering operation to be performed on a second filter that replaces the first filter carried by the filter drawer (2).

13. A device according to any one of claims 1 to 9, characterized in that, in the event that double filtering is necessary for the samples, said device includes a second tank-block (5) and a second filter drawer (2) with a second perforated gasket (7) and a second filter (13) being interposed therebetween, and being disposed in cascade relative to the first tank-block (5) and to the first filter drawer (2), the stand (1) including two further horizontal slideways (23) for enabling the second filter drawer (2) to be inserted therein together with the second tank-block it supports.

14. A device according to any preceding claim, characterized in that the filter drawer (2) is designed to cooperate with a plurality of mutually identical tank-blocks (5) with the number of blocks being a function of the number of samples.

15. A device according to any preceding claim, characterized in that the forced filtering means further include filtrate suction means in communication with the evacuation means (6), and cooperating with the forced filtering means that by apply pressure (17, 18, 19).

16. A device according to any preceding claim, characterized in that said feed device is automatic and programmable and in that the forced filtering means, namely the means for applying pressure and/or suction are themselves also programmable, the assembly being under the control of a controlling computer.

## Patentansprüche

1. Modulare Vorrichtung zum Sammeln, Brüten und Filtrieren von fluidisierten, flüssigen oder gasförmigen Proben, die zu filtrierende Teilchen, wie Bakterien, Zellen oder andere, insbesondere in der Milch oder im Blut enthaltenen Elemente enthalten, wobei die Vorrichtung folgendes aufweist:
- ein Gehäuse (1);
- zumindest einen Filter (13), der in Filtrationsrichtung gesehen zwischen einem stromauf (5) und einem stromab (2) angeordneten Befestigungsblock festgeklemmt ist, welche unter Zwischenschaltung einer perforierten Dichtung (7) aneinander befestigt sind, wobei der stromauf liegende Befestigungsblock (5) eine Anordnung von parallelen Leitungen aufweist, die Schächte (30) zum Speichern von zu behandelnden Proben bilden, wogegen der stromab angeordnete Befestigungsblock (2) in Form einer perforierten Platte ausgebildet ist, und die Schächte (30), die Perforationen der Dichtung (7) und die Perforationen der Platte zueinander so ausgerichtet sind, daß im Bereich des Filters (13) gleich viele unabhängige Filterzonen wie Proben vorliegen;
- Mittel zur Zwangsfiltrierung (17, 19) und
- Mittel zur Evakuierung (6) von Filtraten,
**dadurch gekennzeichnet, daß**
- der stromauf gelegene Befestigungsblock (5) ein unabhängiger Behälterblock ist, der eine Vorbehandlung, wie ein Brüten der in den Schächten (30) enthaltenen Proben ermöglicht, wobei diese Behandlung außerhalb des Gehäuses erfolgt;
- der stromauf gelegene Befestigungsblock (5) außerhalb des Gehäuses (1) durch komplementäre Befestigungsmittel (10, 26), die voneinander lösbar sind, an dem stromab gelegenen Befestigungsblock (2) befestigt ist und
- die Anordnung der zwei Befestigungsblöcke (2, 5) abnehmbar im Inneren des Gehäuses (1) angeordnet ist und durch eine seitliche Öffnung (22) eingebracht wird, um eine Filtration der vorbehandelten Proben durchzuführen, wobei dieses Gehäuse (1) eine obere Öffnung (24) zum Befestigen der Mittel zur Zwangsfiltrierung (17, 18, 19) aufweist.

2. Vorrichtung nach Anspruch 1, **dadurch gekennzeichnet, daß** sie zur Vorbehandlung der Proben in dem Behälterblock (5) außerhalb des Gehäuses (1) an der Grundfläche des Behälterblocks (5) mit zumindest einem abnehmbaren Verschluß für die Schächte (30) versehen ist, wobei der stromab gelegene Befestigungsblock (2) an der Deckfläche des Behälterblocks (5) angeordnet und befestigt ist und die Anordnung der zwei Blöcke (2, 5) vor dem Einbringen in das Gehäuse (1) umgedreht wird.

3. Vorrichtung nach Anspruch 1 oder 2, **dadurch gekennzeichnet, daß** die Anordnung der zwei Blöcke (2, 5) im Inneren des Gehäuses (1) mittels Gleitführungen (23), die in der Einfassung der seitlichen Öffnung (22) des Gehäuses (1) vorgesehen sind, in Form eines Einschubes aufgenommen ist.

4. Vorrichtung nach einem der Ansprüche 1 bis 3, **dadurch gekennzeichnet, daß** die Mittel zur Befestigung der zwei Befestigungsblöcke (2, 5) durch einrastbare Laschen (10) gebildet werden, die mit Nasen (26) zusammenwirken können.

5. Vorrichtung nach einem der Ansprüche 1 bis 4, **dadurch gekennzeichnet, daß** die stromab gelegene Grundfläche des Behälterblocks (5), die stromauf gelegene Deckfläche des Befestigungsblocks (2) und der Filter (13) mit gegengleichen Markierungen versehen sind, die eine Ausrichtung ihrer zugeordneten Perforationen sicherstellt.

6. Vorrichtung nach einem der Ansprüche 1 bis 5, **dadurch gekennzeichnet, daß** die Mittel zur Zwangsfiltrierung (17,18,19) durch Mittel zur Druckbeaufschlagung der in den Schächten (30) des stromauf angeordneten Befestigungsblocks (5) enthaltenen Proben gebildet werden, wobei diese Mittel von einem Kopf (3) gehalten werden, der einen festen (25) und einen bewegbaren (14) Bestandteil aufweist, welcher eine Kammer zur Druckerzeugung (17) umfaßt, deren Boden von einer Mehrzahl von kalibrierten Röhren (20) durchsetzt ist, die teilweise in die Schächte (30) des stromauf gelegenen Befestigungsblocks (5) hineinragen und mit einer in der Decke (29a) der Kammer (17) vorgesehenen Druckluftzufuhr (19) in Verbindung stehen, wobei der Außendurchmesser der kalibrierten Röhren (20) im wesentlichen gleich dem Innendurchmesser der Schächte (30) ist und der bewegliche Teil (14) mit Hilfe eines Betätigungsmittels, wie einem Heber (4), bewegbar ist.

7. Vorrichtung nach Anspruch 6, **dadurch gekennzeichnet, daß** die Decke (29a) der Kammer (17) zur Druckerzeugung von einer Mehrzahl von Röhren (18) durchsetzt ist, die durch das Innere dieser Kammer (17) führen, wobei diese Röhren (18) mit einer Versorgungseinrichtung zum Einbringen von Reaktionsagentien verbunden sind.

8. Vorrichtung nach einem Anspruch 7, **dadurch gekennzeichnet, daß** die Mittel zum Verschließen der Schächte (30) des stromauf gelegenen Befestigungsblocks (5) durch eine durchströmbare anhaftende Membran gebildet werden und daß die kalibrierten Röhren (20) der Mittel zur Zwangsfiltrierung (17, 18, 19) abgeschrägt sind, um diese Membran zu durchdringen.

9. Vorrichtung nach einem der Ansprüche 1 bis 8, **dadurch gekennzeichnet, daß** die Mittel zur Evakuierung (6) durch einen Behälter gebildet werden, der an dem unteren Teil des Gehäuses (1) angeordnet ist, wobei der stromab gelegene Befestigungsblock (2) dichtend an diesem Behälter aufliegt, wenn er in das Gehäuse (1) eingebracht ist.

10. Vorrichtung nach Anspruch 9, **dadurch gekennzeichnet, daß** der Behälter zur Evakuierung mit einer Öffnung (8) zusammenwirkt, die in der Grundfläche des Gehäuses (1) vorgesehen ist.

11. Vorrichtung nach einem der vorigen Ansprüche, **dadurch gekennzeichnet, daß** die Vorrichtung zu beiden Seiten der perforierten Dichtung (7) je ein Filter (13) mit unterschiedlicher Durchlässigkeit aufweist, falls eine Doppelfiltrierung der Probe erforderlich ist, wobei der Filter mit der geringeren Porengröße stromab angeordnet ist.

12. Vorrichtung nach einem der Ansprüche 1 bis 9, **dadurch gekennzeichnet, daß** die Mittel zur Evakuierung (6) abnehmbar sind und durch einen Block gebildet werden, der mit dem Behälterblock (5) gleichartig ist und ein abnehmbares Mittel zum Verschließen seiner stromab gelegenen Grundfläche aufweist, falls eine Doppelfiltrierung erforderlich ist, wobei diese einen Behälter bildenden Mittel zur Evakuierung dazu geeignet sind, den stromauf angeordneten Behälterblock (5) zu ersetzen und nach der ersten Filtrierung in der Weise durch herkömmliche Mittel zur Evakuierung (6) ersetzt werden, daß die zweite Filtrierung durch einen zweiten Filter erfolgt, welcher den ersten, von dem Filtereinschub (2) gehaltenen Filter ersetzt.

13. Vorrichtung nach einem der Ansprüche 1 bis 9, **dadurch gekennzeichnet, daß** sie einen zweiten Behälterblock (5) und einen zweiten Filtereinschub (2) aufweist, zwischen welchen eine zweite perforierte Dichtung (7) und ein zweiter Filter (13) angeordnet sind und die bezüglich des ersten Behälterblocks (5) und des ersten Filtereinschubes (2) kaskadenartig angeordnet sind, falls für eine Doppelfiltrierung der Proben erforderlich ist, wobei das Gehäuse (1) zwei weitere horizontale Gleitführungen (23) aufweist, in welchen der zweiten Filtereinschub (2) mit dem durch diesen gehaltenen zweiten Behälterblock eingeschoben werden kann.

14. Vorrichtung nach einem der vorigen Ansprüche, **dadurch gekennzeichnet, daß** der Filtereinschub (2) mit einer Mehrzahl von untereinander gleichartigen Behälterblöcken (5) zusammenwirken kann, deren Anzahl von der Anzahl der Proben abhängig ist.

15. Vorrichtung nach einem der vorigen Ansprüche, **dadurch gekennzeichnet, daß** die Mittel zur Zwangsfiltrierung ebenso Mittel zum Ansaugen von Filtraten aufweisen, welche mit den Mittein zur Evakuierung (6) in Verbindung stehen und durch Druckbeaufschlagung mit den Mitteln zur Zwangsfiltrierung (17,18,19) zusammenwirken.

16. Vorrichtung nach einem der vorigen Ansprüche, **dadurch gekennzeichnet, daß** sie automatisch und programmierbar betrieben werden kann und daß die Mittel zur Zwangsfiltrierung, das heißt, die Druckbeaufschlagung und/oder das Ansaugen, ebenso programmierbar sind, wobei die Anordnung durch einen Steuerrechner angesteuert wird.
